# EUROPEAN PATENT APPLICATION

(11) **EP 4 461 145 A1**
(43) Date of publication of application: **13.11.2024**
(21) Application number: 23216570.4
(22) Date of filing: 14.12.2023
(51) Int. Cl.: A23L 33/135, C12N 1/20

(54) **LACTIC ACID BACTERIAL STRAIN, COMPOSITION FOR IMPROVING GUT MICROBIOTA COMPOSITION, PRODUCT OF SAID LACTIC ACID BACTERIAL STRAIN, AND METHOD FOR THE PREPARATION OF THE COMPOSITION THEREOF**

(30) Priority: 10.05.2023 TW 112117251; 08.11.2023 TW 112143096
(71) Applicant: Sunway Biotech Co., Ltd., Taipei City 114, (TW)
(72) Inventor: PAN, TZU-MING, 106 Taipei City (TW); SHIH, TSUNG-WEI, 114 Taipei City (TW); HSU, WEI-HSUAN, 709 Tainan City (TW)
(74) Representative: Kurig, Thomas

(57) **Abstract**

The main objective of the present invention is to provide a lactic acid bacterial strain and a composition for improving gut microbiota composition. The composition comprises the lactic acid bacterial strain and/or extracellular vesicles secreted by the lactic acid bacterial strain. Another objective of the present invention is to provide products of the lactic acid bacterial strain and a method for the preparation of the composition. Additionally, the composition of the present invention has the capability to influence the growth of *Firmicutes* and *Bacteroidetes,* thereby leading to an improvement in gut microbiota composition.

## Description

### BACKGROUND OF THE INVENTION

### 1. Field of the Invention

The present invention relates to the technical field of improving gut microbiota composition, particularly the technical field of a composition containing a novel lactic acid bacterial strain and/or the extracellular vesicles secreted by said novel lactic acid bacterial strain for improving gut microbiota composition.

### 2. Description of the Prior Art

The composition and changes of gut microbiota are associated with human health. There is a complex nervous system in the gut. The gut and brain are interconnected and influenced by the gut-brain axis. Microorganisms in the gut produce metabolites and neurotransmitters that affect intestinal cells and immune cells, which in turn affect the host's gastrointestinal function and even behavioral and emotional performance.

Gut microbiota contains a variety of symbiotic microorganisms, which are mainly composed of bacteria, mostly obligate anaerobes, and there are four major phyla: *Bacteroidetes, Firmicutes, Actinobacteria,* and *Proteobacteria.* In addition, it was found that the *Firmicutes* to *Bacteroidetes* (F/B) ratio varies according to diseases, with healthy individuals having a large number of *Bacteroidetes* in their gut flora and patients with disease having a large number of *Firmicutes* in their gut flora. Obese patients have a significant reduction in the abundance of the phylum *Bacteroidetes* in their guts, which includes many beneficial gut bacteria. *Bacteroides thetaiotaomicron* is relatively low in the gut of obese patients and is associated with obesity-related metabolites and hormonal changes, so it has been identified as a key microorganism associated with the disease and is highly feasible to be used as a target for treatment. Meanwhile, gut microorganisms play an important role in maintaining the stability of the gut and the integrity of the mucosal immune system, and they help maintain the integrity of the gut and regulate the permeability of the intestinal barrier. Furthermore, evidence has shown that dysbiosis and changes in microbiota are associated with many diseases and dysfunctions, such as obesity, inflammatory bowel disease (IBD), autism, and neurodegenerative diseases, and that specific gut microorganisms are altered with varying degrees of diseases (Liu et al. Nature Medicine 2017, 23, 859-868; Hall et al. Genome Medicine 2017, 9, 103; Finegold et al. Anaerobe 2010, 16, 444-453; Scheperjans et al. Movement Disorders 2015, 30, 350-358).

Currently, the regulation of gut microbiota is mostly achieved through the direct consumption of probiotics, which can increase the population of beneficial bacteria and suppress the growth of harmful bacteria through colonization. At present, there is also a method called faecal microbiota transplant (FMT), which involves transferring healthy gut microbiota to the digestive system of a host.

However, probiotics generally have the problem of not being able to colonize, making the impact on the microbiota limited. Moreover, FMT treatment still has many drawbacks and safety concerns. Currently, the main routes of faecal microbiota transplantation include the upper, middle and lower gastrointestinal tracts. The faecal microbiota transplantation through the upper gastrointestinal tract is done by oral administration of faecal microbiota, but the disadvantage is that the microorganisms are easily affected by bile salts. The faecal microbiota transplantation through the middle gastrointestinal tract is performed by a nasogastric tube or PEG tube, but the disadvantage is that accidental aspiration and reflux may occur if not done properly, and the microorganisms are also affected by bile salts. The faecal microbiota transplantation through the lower gastrointestinal tract is performed by colonoscopy, enema, or transendoscopic enteral tubing, but the disadvantage is that the faecal microbiota can only cover the sigmoid colon and multiple faecal microbiota transplants are required. Although FMT was previously considered a safe treatment, recent clinical reports and cases reported by the US Food and Drug Administration have found that a patient died after receiving faecal microbiota from a healthy donor, whose faecal microbiota contained multidrug-resistant genes in *Escherichia coli.* In addition, current technology is still unable to effectively evaluate the antibiotic-resistance genes in the microbiota of a healthy FMT donor, thus FMT still carries potential risks.

In consideration of the limitations and drawbacks of the current method for regulation of gut microbiota and the fact that the composition and changes of gut microbiota are closely associated with human health and many diseases, the inventors of the present application hope to use extracellular vesicles (EV), which are lipid bilayer-delimited nanoparticles containing nucleic acids and proteins inside, to safely and effectively regulate the composition and changes of gut microbiota by interacting with neighboring bacteria. Given this, the inventors of the present application have made great efforts to research and have finally completed the development of a novel lactic acid bacterial strain, compositions for improving gut microbiota composition, products of said lactic acid bacterial strain, and a method for the preparation of the composition. In addition, the efficacy of improving gut health can be achieved through small amounts of extracellular vesicles secreted by microorganisms.

### SUMMARY OF THE INVENTION

The primary object of the present invention is to provide a lactic acid bacterial strain and a composition for improving the gut microbiota composition, in which the composition comprises said lactic acid bacterial strain and/or extracellular vesicles secreted by said lactic acid bacterial strain. In addition, the composition for improving gut microbiota composition may be administered by oral route.

In line with the objectives previously described, the present invention provides a novel lactic acid bacterial strain, wherein said lactic acid bacterial strain is a *Pediococcus acidilactici,* deposited at the NCIMB Ltd., with an Accession number of NCIMB 44102.

Furthermore, the present invention provides a composition for improving gut microbiota composition, comprising an effective amount of extracellular vesicles secreted by a lactic acid bacterial strain, wherein said lactic acid bacterial strain is a *Pediococcus acidilactici,* deposited at the NCIMB Ltd., with an Accession number of NCIMB 44102.

In the above-mentioned composition, said effective amount of said extracellular vesicles is at least 10⁸ particles/ml, and said extracellular vesicles are selected from the group consisting of exosomes, microvesicles, ectosomes, and apoptotic bodies.

In addition, the above-mentioned composition is effective in improving the gut microbiota composition that affects the progression of Alzheimer's disease (AD).

In one aspect, said extracellular vesicles of the above-mentioned composition can affect the growth of *Firmicutes* and/or *Bacteroidetes.*

In another aspect, said extracellular vesicles can affect the growth of at least one bacterial class selected from the group consisting of *Bacteroidia, Clostridia,* and *Bacilli.*

In another aspect, said extracellular vesicles can affect the growth of at least one bacterial family selected from the group consisting of *Lactobacillaceae, Muribaculaceae, Lachnospiraceae, Clostridiaceae, Desulfovibrionaceae, Erysipelotrichaceae, Eggerthellaceae, Akkermansiaceae, Ruminococcaceae,* and *Eubacteriaceae.* Further, said extracellular vesicles can affect the growth of *Muribaculum* and/or *Lachnospira.*

In yet another aspect, the composition as described above, said extracellular vesicles therein may facilitate the growth of *Lactobacillus acidophilus.*

In one embodiment, the invention provides a nutritional supplement comprising a *Pediococcus acidilactici,* deposited at the National Collection of Industrial, Food and Marine Bacteria (NCIMB Ltd.), with an Accession number of NCIMB 44102.

In one embodiment, the invention provides a food product comprising a *Pediococcus acidilactici,* deposited at the NCIMB Ltd., with an Accession number of NCIMB 44102.

In one embodiment, the invention provides a dietary supplement comprising a *Pediococcus acidilactici,* deposited at the NCIMB Ltd., with an Accession number of NCIMB 44102.

In one embodiment, the invention provides a food additive comprising a *Pediococcus acidilactici,* deposited at the NCIMB Ltd., with an Accession number of NCIMB 44102.

In one embodiment, the invention provides a pharmaceutical composition comprising a *Pediococcus acidilactici,* deposited at the NCIMB Ltd., with an Accession number of NCIMB 44102.

In one embodiment, the invention provides a feedstuff comprising a *Pediococcus acidilactici,* deposited at the NCIMB Ltd., with an Accession number of NCIMB 44102.

Another object of the present invention is to provide a method for the preparation of a composition using a lactic acid bacterial strain and/or extracellular vesicles secreted by the lactic acid bacterial strain, wherein said lactic acid bacterial strain is a *Pediococcus acidilactici,* deposited at the NCIMB Ltd., with an Accession number of NCIMB 44102.

Yet another object of the present invention is to provide an application of a composition for improving gut microbiota composition in various forms, such as food, beverages, health food, additives, and medical compounds. This application allows for easy everyday use and consumption, enabling ordinary people to maintain gut health daily.

### BRIEF DESCRIPTION OF THE DRAWINGS

FIG. 1A shows an image of the extracellular vesicle of the lactic acid bacterial strain according to the present invention as observed by transmission electron microscope (TEM);
FIG. 1B shows the average particle size distribution of the extracellular vesicle of the lactic acid bacterial strain according to the present invention;
FIG. 2 shows the relative abundance of gut microorganisms in *APP*^{*NL-G-F*/*NL-G-F*} transgenic mice after oral administration of the extracellular vesicles secreted by the lactic acid bacterial strain according to the present invention;
FIG. 3A and 3B show the growth curve of the *Lactobacillus acidophilus* co-cultured with the extracellular vesicles secreted by the lactic acid bacterial strain according to the present invention;
FIG. 4A, 4B, 4C, and 4D show the growth curve of gut bacteria, such as *Lachnospiraceae sp.* and *Ruminococcaceae sp.,* co-cultured with the extracellular vesicles secreted by the lactic acid bacterial strain according to the present invention;
FIG. 5 shows fluorescence staining images of mouse faecal microorganisms reacted with the extracellular vesicles secreted by the lactic acid bacterial strain according to the present invention;
FIG. 6A shows the analysis results of mouse gut microbiota without the addition of the extracellular vesicles secreted by the lactic acid bacterial strain according to the present invention, analyzed by flow cytometer;
FIG. 6B shows the analysis results of mouse gut microbiota with the addition of the extracellular vesicles secreted by the lactic acid bacterial strain according to the present invention, analyzed by flow cytometer;
FIG.7 is a bar chart that shows the relative abundance of gut microorganisms in mice ingesting the extracellular vesicles secreted by the lactic acid bacterial strain according to the present invention;
FIG. 8 is the phylogenetic tree of *Pediococcus acidilactici;*
FIG. 9 shows the gel electrophoresis image of the amplified products using CGPA01_1590-F/R primers; and
FIG. 10 shows the gel electrophoresis image of the amplified products using MA18/5M_850-F/R primers.

### DETAILED DESCRIPTION OF THE PREFERRED EMBODIMENTS

Unless otherwise defined, all terms (including technical and scientific terms) used herein have the same meaning as commonly understood by one of ordinary skill in the art to which present inventive concepts belong. The present invention will be illustrated in more detail with the following exemplary embodiments; however, the present invention is not limited by these exemplary embodiments. Unless otherwise specified, the materials used in the present invention are commercially available and the following are only examples of commercially available routes.

The present invention provides a novel lactic acid bacterial strain and a composition for improving gut microbiota composition. The composition comprises the lactic acid bacterial strain and/or extracellular vesicles secreted by the lactic acid bacterial strain, wherein the lactic acid bacterial strain is deposited with the Accession number of NCIMB 44102 at the NCIMB Ltd. (National Collection of Industrial, Food and Marine Bacteria) in the United Kingdom. The inventors of the present application showed the novel use or function of the extracellular vesicles secreted by *Pediococcus acidilactici* in improving gut microbiota composition through a transgenic mouse model of Alzheimer's disease (knock-in of the amyloid beta precursor protein gene (*APP* gene)).

The *Pediococcus acidilactici* according to the present invention is a novel lactic acid bacterial strain isolated from the guts of chickens by the inventors of the present application in the laboratory through screening.

The extracellular vesicles secreted by the *Pediococcus acidilactici* of the present invention were found to be effective in improving the abundance of gut microbiota in mice through analysis of mouse animal models. Specifically, said extracellular vesicles could effectively improve gut microbiota composition in Alzheimer's transgenic mice, restoring gut microbiota composition in said Alzheimer's transgenic mice to that of a healthy state.

Furthermore, the aforementioned isolated lactic acid bacterial strain comprises progeny after subculture or mutation strains thereof possessing the same bacteriological characteristics, genomic features, or uses (for improving gut microbiota composition) as described in the present invention.

The composition described herein refers to forms suitable to the application of the present invention and may include, but not limited to, nutritional supplement, food product, dietary supplement, food additive, pharmaceutical compositions for animals and human beings, feedstuff, beverages, health foods, additives in animal drinking water, animal feed additives, beverage additives and the like.

The term "improving" means, compared with those that do not use the *Pediococcus acidilactici* of the present application and/or the extracellular vesicles thereof or composition containing the *Pediococcus acidilactici* of the present application and/or the extracellular vesicles thereof, one that uses the *Pediococcus acidilactici* of the present application and/or the extracellular vesicles thereof or composition containing the *Pediococcus acidilactici* of the present application and/or the extracellular vesicles thereof can improve the composition of the gut microbiota.

The term "effective amount' means an amount of active ingredient that can improve, treat, or recover one or more gut microorganisms; it may be referred to as "treating-effective amount" or "improving-effective amount". In addition, the term "pharmaceutically acceptable" means substances used in the composition must be compatible with other components in the formulation and be harmless to the subject.

The composition of the present invention can be prepared into a dosage form suitable for the application of the composition of the present invention by using a conventional technique well-known to one skilled in the art through formulating the above-described *Pediococcus acidilactici* isolated strain and/or the extracellular vesicles thereof with a pharmaceutically acceptable vehicle. The dosage form may include, but not limited to, solution, emulsion, suspension, powder, tablet, pill, lozenge, troche, capsule, and other suitable forms.

In the aforementioned composition, one or more dissolving aids, buffers, storage agents, colorants, fragrances, flavoring agents, excipients, and the like commonly used in the pharmaceutical field can be added as desired.

In another preferred embodiment, the composition of the present invention can be further added into an edible material to prepare a food or health product. The edible material may include, but not limited to: water; fluid milk product; milk; concentrated milk; fermented milk, such as yogurt, frozen yogurt, sour milk, lactic fermenting beverage; milk powder; ice cream; cream cheese; dry cheese; soybean milk; fermented soybean milk; fruit and vegetable juice; juice; sports drink; confectionery; jelly; baby food; health food; animal feed; herbal medicine; dietary supplement, and the like.

Further, the present invention also provides a method for improving gut microbiota composition by administering an effective amount of the aforementioned composition to a patient with an intestinal disease or Alzheimer's disease for improving gut microbiota composition.

In addition, the present invention also provides a method or use of the aforementioned *Pediococcus acidilactici* and/or the extracellular vesicles secreted by the *Pediococcus acidilactici* for the preparation of compositions that improve the gut microbiota composition.

The composition of the present invention and the method using the same for improving gut microbiota composition are not limited to a specific administration route and can be adjusted according to needs. However, the preferred administration route is oral administration with an appropriate dosage form.

### Sources of the Strains

The *Lactobacillus acidophilus* used in the following embodiments of the present invention is a standard strain: *Lactobacillus acidophilus BCRC 14079,* purchased from the Bioresource Collection and Research Center (BCRC) of Food Industry Research and Development Institute (FIRDI) in Hsinchu, TAIWAN. The *Lachnospiraceae sp.* and *Ruminococcaceae sp.* used in the present invention are standard strains: *Lachnospiraceae sp.* TSD-26 and *Ruminococcaceae sp.* TSD-27, purchased from the American Type Culture Collection (ATCC). The novel lactic acid bacterial strain of the present invention is *Pediococcus acidilactici,* which was isolated through the screening of chicken intestines and is deposited at the NCIMB Ltd. with an Accession number of NCIMB 44102.

### Experimental Animals

The animal strain used in the following embodiments of the present invention was an *APP*^{*NL-G-F*/*NL-G-F*} transgenic mouse (TG), which was a knock-in AD model using the amyloid beta precursor protein gene (*APP* gene). The transgenic mouse carried the Swedish double mutation (KM670/671NL) and the Iberian mutation (I716F) in the Aβ sequence, increasing Aβ production and enhancing the Aβ42/40 ratio. The transgenic mouse began to show memory deficits at the age of 6 months, and the control group used a C57BL/6 mouse (non-transgenic mouse; non-TG) as a control (Nilsson et al. ACS Chemical Neuroscience 2014, 5, 499-502).

### Preparation of Extracellular Vesicles

First, the *Pediococcus acidilactici* of the present invention was cultured in MRS medium at 37°C for 24 hours. The bacterial cells were removed by centrifugation at 10,000 ×g for 30 minutes, and the supernatant was filtered. The filtrate was then subjected to ultracentrifugation at 247,537 ×g at 4°C, and the supernatant was removed. Next, the precipitate was washed with Dulbecco's phosphate buffered saline (DPBS) and the resulting suspension was filtered through a 0.22 µm sterilization membrane. The filtrate was then centrifuged at 247,537 × g at 4 °C and the supernatant was removed. Finally, the precipitate was resuspended in DPBS to obtain the extracellular vesicles and the obtained extracellular vesicles were stored at -80 °C (Choi et al. Experimental Neurobiology 2019, 28, 158-171).

### Embodiment 1: Morphological characteristics of the extracellular vesicles secreted by Pediococcus acidilactici of the present invention

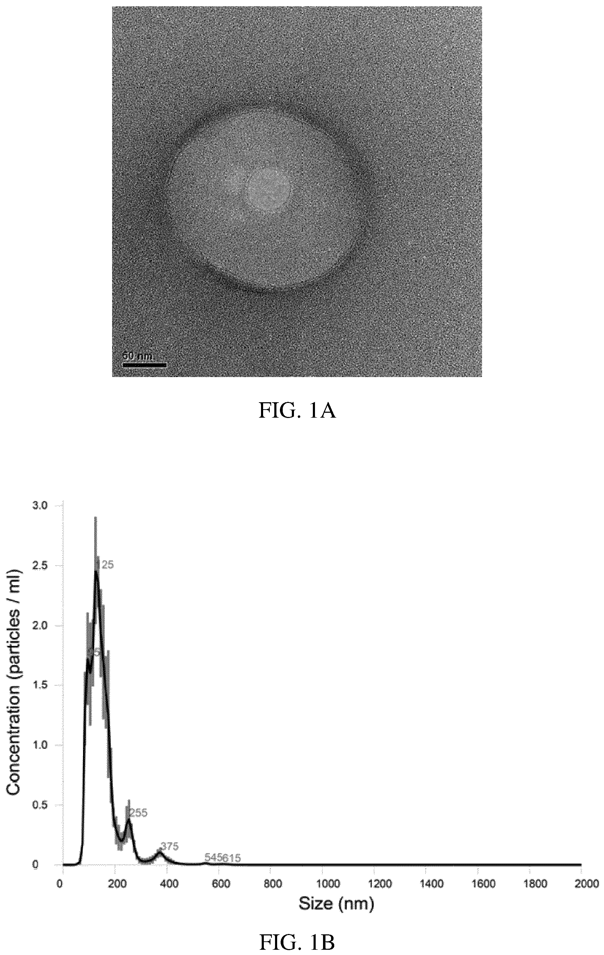

In the present embodiment, the culture medium of the *Pediococcus acidilactici* of the present invention was centrifuged by ultracentrifugation to purify the extracellular vesicles (extracellular vesicles derived from lactic acid bacteria, hereinafter referred to as LAB-EV) thereof, and the morphological characteristics of the purified LAB-EV were observed by a transmission electron microscope. As shown in FIG. 1A, the extracellular vesicle exhibited a spherical lipid bilayer structure of nanometer size. In addition, the LAB-EV were properly diluted with Dulbecco's phosphate buffered saline (DPBS) and further analyzed by a nanoparticle tracking analyzer. The diluted liquid was irradiated with a laser light source and the Brownian motion of the scattered light nanoparticles was observed through a microscope to calculate the average particle size and particle number (quantitative analysis) of the LAB-EV sample. The average particle size distribution of the LAB-EV is shown in FIG. 1B, and the average particle size of the LAB-EV is approximately 125 nanometers (nm).

### Embodiment 2: Relative abundance of the microorganisms in the gut microbiota of transgenic mice after oral administration of extracellular vesicles secreted by the Pediococcus acidilactici of the present invention

In the present embodiment, mice were orally administered with LAB-EV in a single daily dose, and after 4 weeks of LAB-EV administration, the mice were sacrificed and the relative abundance of the microorganisms in the gut microbiota of the mice was analyzed.

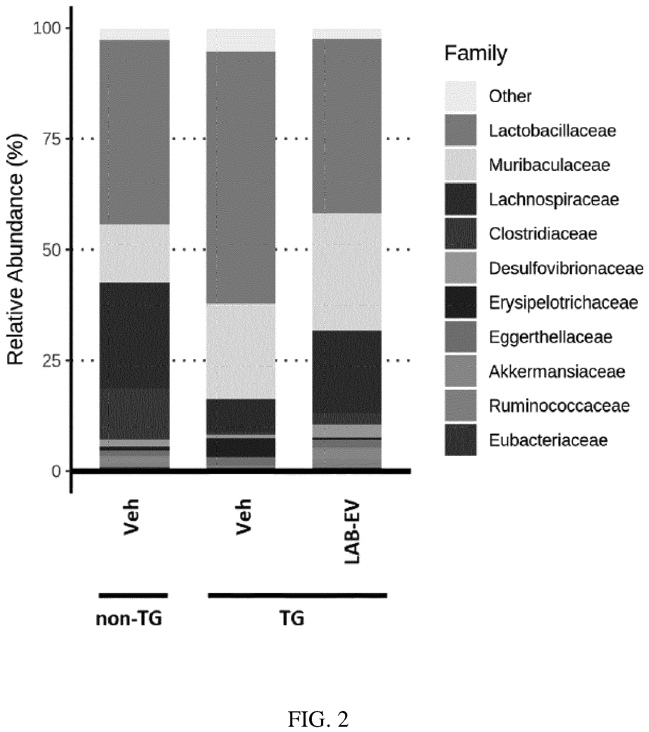

FIG. 2 shows the relative abundance of the microorganisms in the gut microbiota of mice after oral administration of LAB-EV of the present invention, wherein Non-TG Veh denotes non-transgenic mice orally administered with the vehicle, n=6, TG Veh denotes transgenic mice (knock-in of the amyloid beta precursor protein gene (*APP* gene)) orally administered with the vehicle, n=6, TG LAB-EV denotes transgenic mice (knock-in of *APP* gene) orally administered with LAB-EV, n=6. Here Veh (Vehicle) refers to DPBS, which is the solvent of LAB-EV

The results in FIG. 2 show the top 10 bacterial families in the relative abundance of mouse gut microorganisms, and those ranked outside the top 10 are classified as "others", among which the top 10 families include: *Lactobacillacea, Muribaculaceae, Lachnospiraceae, Clostridiaceae, Desulfovibrionaceae, Erysipelotrichaceae, Eggerthellaceae, Akkermansiaceae, Ruminococcaceae,* and *Eubacteriaceae.* In addition, the results in FIG. 2 show that there are differences in the gut microbiota composition between non-TG Veh mice and TG Veh mice at the family level, while the gut microbiota composition in TG LAB-EV mice is closer to that of non-TG Veh mice. This indicates that gut microbiota composition in the transgenic mice is improved after the administration of LAB-EV, which also indicates that the gut microbiota composition of the transgenic mice is restored to that of healthy mice. Therefore, LAB-EV can effectively improve the gut microbiota of the transgenic mice and restore the gut microbiota composition in the transgenic mice to that of healthy mice.

### Embodiment 3: Co-culture of Lactobacillus acidophilus with LAB-EV secreted by the Pediococcus acidilactici of the present invention

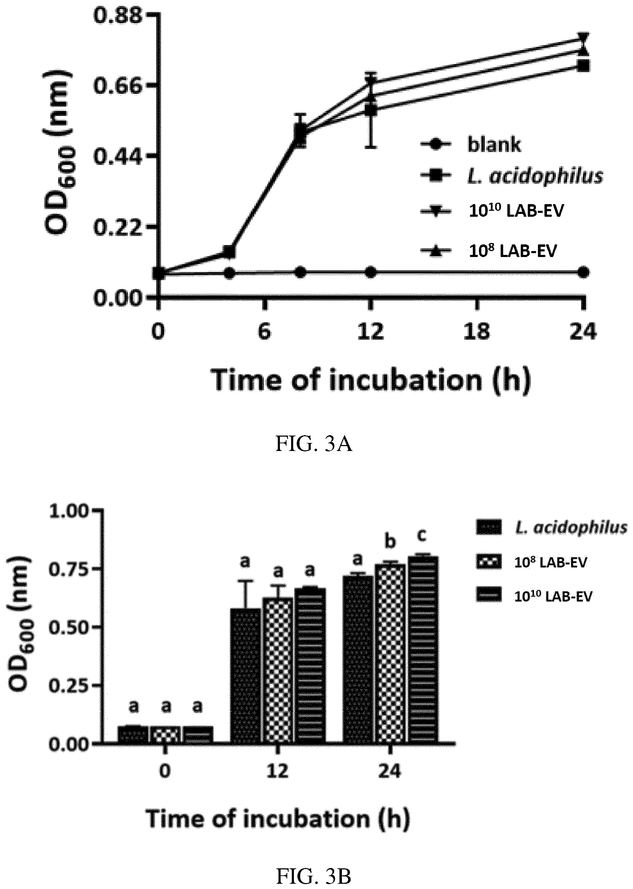

In the present embodiment, an in vitro experiment was conducted by co-culturing LAB-EV with *Lactobacillus acidophilus* to analyze whether the LAB-EV of the present invention has the potential to promote the growth of *Lactobacillus acidophilus.* LAB-EV and *Lactobacillus acidophilus* were co-cultured, and the absorbance values at wavelengths of 600 nm were measured at different time points. Finally, broken line graphs and histograms were obtained as shown in FIGS. 3A and 3B, respectively, wherein the data were presented as the mean±standard error of the mean (SEM) for three replicates and analyzed using one-way analysis of variance (One-way ANOVA), followed by multiple group comparisons using Tukey's test. Significant differences were denoted by different letters.

The results shown in FIGS 3A and 3B show that LAB-EV promoted the growth of *Lactobacillus acidophilus,* and the higher concentration of LAB-EV promoted the growth of *Lactobacillus acidophilus* better, that is, the effect of LAB-EV on the growth of *Lactobacillus acidophilus* was dose-dependent. For example, the growth of *Lactobacillus acidophilus* was increased significantly when co-cultured with LAB-EV at a concentration of 10¹⁰ particles/mL, while the growth of *Lactobacillus acidophilus* was increased less effectively when co-cultured with LAB-EV at a concentration of 10⁸ particles/mL.

### Embodiment 4: Co-culture of LAB-EV of the present invention and gut bacteria

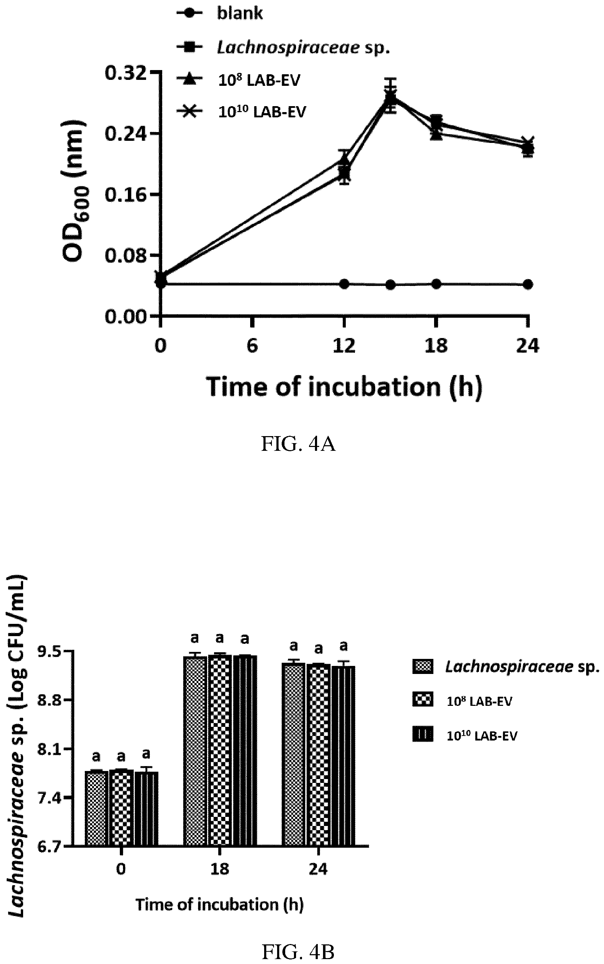

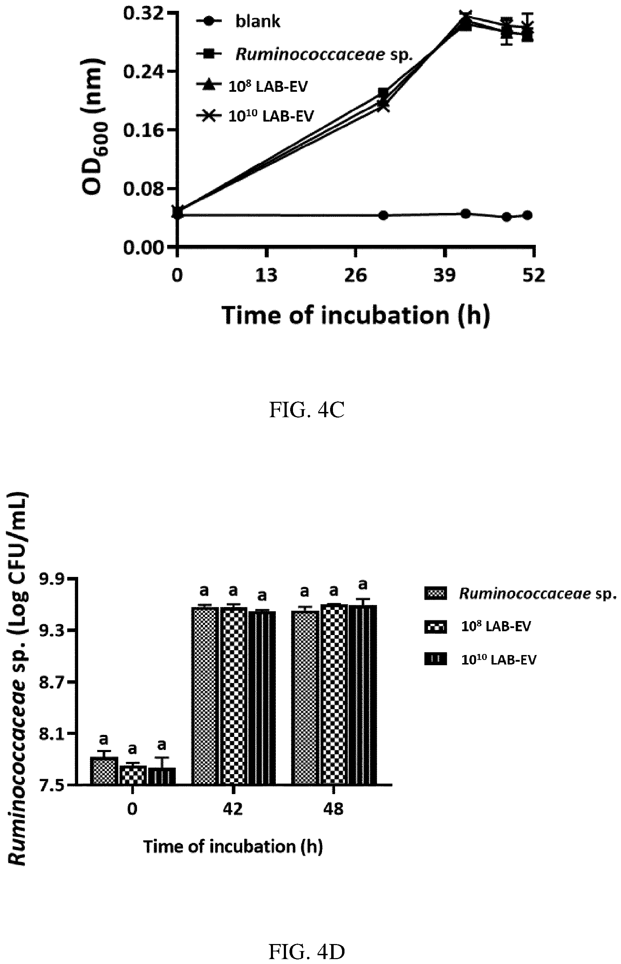

In the present embodiment, LAB-EV was co-cultured respectively with *Lachnospiraceae sp.* and *Ruminococcaceae sp.* The absorbance values at wavelength 600 nm were measured at different time points, and the results were represented as the broken line graphs in FIG. 4A and FIG. 4C, respectively. The bacteria count was performed at different time points by plate counting (Log CFU/mL), and the results were presented by histograms as shown in FIG. 4B and FIG. 4D, wherein the data were presented as the mean±standard error of the mean (SEM) for three replicates and different letters denoted significant differences (p < 0.05).

The results shown in FIGS. 4A, 4B, 4C, and 4D indicate that LAB-EV had no effect on the growth of both gut bacteria *Lachnospiraceae sp.* and *Ruminococcaceae sp.* Moreover, neither the higher nor the lower concentration of LAB-EV had any effect on the growth of both gut bacteria *Lachnospiraceae sp.* and *Ruminococcaceae sp.*

Embodiment 5: Fluorescence staining of mouse faecal microorganisms reacted with LAB-EV

In order to investigate whether gut microorganisms can uptake LAB-EV and to determine the types of gut bacteria that can uptake LAB-EV, in the present embodiment, 10⁸ particles/ml of LAB-EV that were stained with CFSE (green) and mouse faecal bacteria that were stained with DRAQ5 (red) were added to a well plate. The CFSE had an excitation wavelength of 492 nm and an emission wavelength of 517 nm, and the DRAQ5 had an excitation wavelength of 647 nm and an emission wavelength of 665 nm. CFSE is a staining dye of carboxyfluorescein succinimidyl ester and DRAQ5 was purchased from Abcam, Bristol, UK.

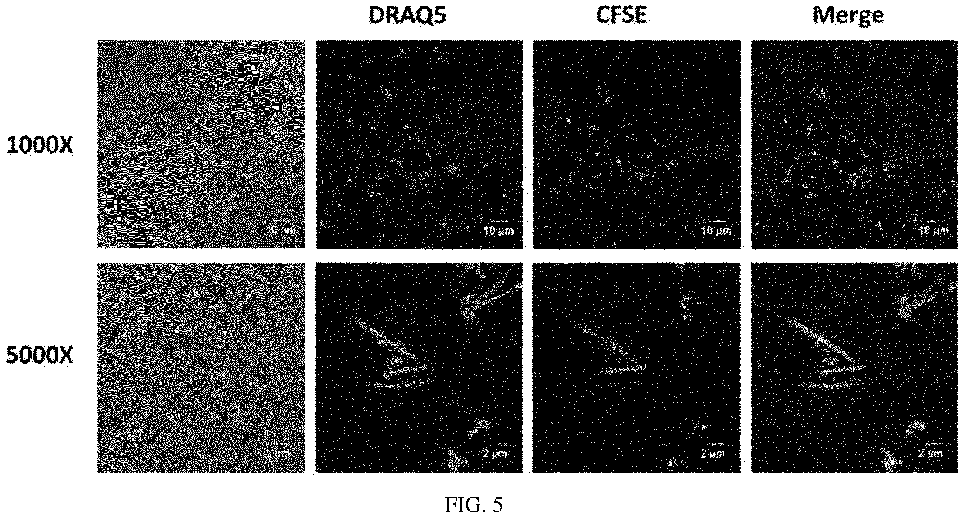

According to the results shown in FIG. 5, the red fluorescence represents the bacterial cells in mouse faeces stained with DRAQ5, in which the bacterial cells are distributed in rod-like or spherical shapes, and the green fluorescence represents the LAB-EV stained with CFSE. The overlap of both shows yellow fluorescence. The experiment results show that LAB-EV can be taken up by gut microorganisms, which are composed of a variety of bacteria. Some bacterial cells do not overlap with green fluorescence, indicating that not all types of bacteria take up LAB-EV, and only specific bacteria take up LAB-EV

### Embodiment 6: LAB-EV can be taken up by gut bacteria in mice

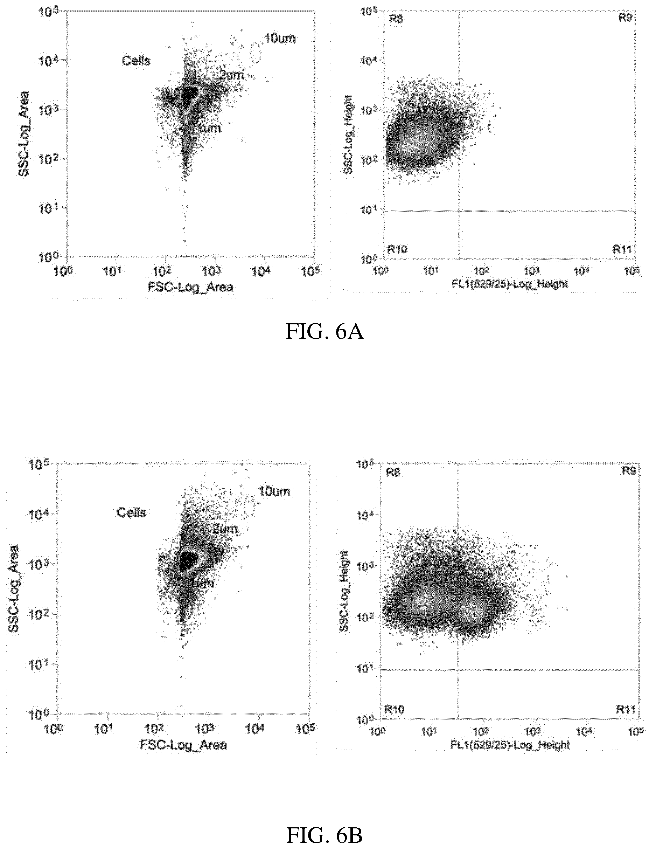

In the present embodiment, mouse gut microorganisms without the addition of LAB-EV (FIG. 6A) and mouse gut microorganisms with the addition of LAB-EV (FIG. 6B) were analyzed by a flow cytometer, wherein the mouse gut microorganisms with the addition of LAB-EV refers to the incubation of CFSE-stained LAB-EV at a quantity of 10¹⁰ particles with the mouse gut bacteria at 37°C in an anaerobic environment, and n=6 . CFSE is a staining dye of carboxyfluorescein succinimidyl ester.

According to the results shown in FIG. 6A, mouse gut microorganisms without the addition of LAB-EV were analyzed by flow cytometry and specific regions of bacteria were selected for analysis. It can be found that the selected regions contain cells of 1 µm, 2 µm, and 10 µm in size. In addition, in the quadrant plot, the majority of the gut microorganisms fall in the R8 quadrant and account for approximately 98.58% of the total microbiota.

According to the results shown in FIG. 6B, mouse gut microorganisms with the addition of LAB-EV were analyzed by a flow cytometer and regions with bacteria were selected for analysis. It can be found that the selected regions also contain cells of 1 µm, 2 µm, and 10 µm in size. In the quadrant plot, it can be found that in addition to the majority of cells falling within the R8 quadrant, 32.31% of cells also fall within the R9 quadrant.

From the above results, it can be found that the cell sizes of the selected regions were 1 µm, 2 µm and 10 µm, and these lengths correspond to the sizes of the bacteria. Furthermore, by analyzing the mouse gut microbiota without the addition of LAB-EV as a control group, the range of gut bacterial populations in mice can be determined. Moreover, by adding fluorescently labeled LAB-EV into the mouse gut microbiota, fluorescence signals were observed, indicating that some microorganisms in the mouse gut had taken up LAB-EV It was observed that approximately 32.31% of bacteria in the mouse gut microbiota had taken up LAB-EV

### Embodiment 7: Determining the types of gut microorganisms in mice that take up LAB-EV

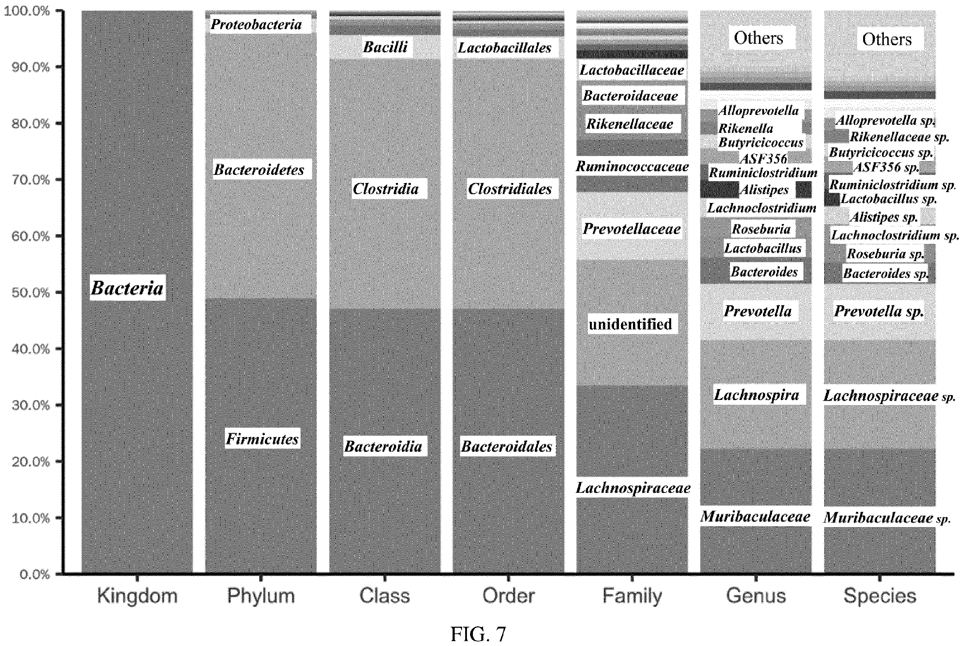

In the present embodiment, the gut microorganisms that had taken up LAB-EV in embodiment 6 were chosen and sequenced. As shown in FIG. 7, after sequencing the gut microorganisms that reacted with LAB-EV, a total of 1 kingdom, 9 phyla, 14 classes, 19 orders, 37 families, 74 genera, and 87 species of gut microorganisms were identified. The relative abundance bar chart in FIG. 7 shows the proportion of bacteria in different taxonomic groups, all of which are in the bacterial kingdom, with the majority being *Firmicutes* and *Bacteroidetes,* accounting for 49% and 47% respectively. At the class level, *Bacteroidia* accounts for 47%, *Clostridia* accounts for 44%, and *Bacilli* accounts for 4%. At the family level, *Lachnospiraceae* is the most abundant and accounts for 33%. The two most abundant genera and species are *Muribaculum*/*Muribaculum sp.* (22%) and *LachnospiralLachnospira sp.* (19%), respectively.

For easy comparison, the relative abundance bar chart in FIG. 7 contains the following categories of microorganisms listed in order: *Bacteria; Proteobacteria, Bacteroidetes, Firmicutes; Bacilli, Clostridia, Bacteroidia; Lactobacillales, Clostridiales, Bacteroidales; Lactobacillaceae, Bacteroidaceae, Rikenellaceae, Ruminococcaceae, Prevotellaceae, Lachnospiraceae; Alloprevotella, Rikenella, Butyricicoccus, ASF356, Ruminiclostridium, Alistipes, Lachnoclostridium, Roseburia, Lactobacillus, Bacteroides, Prevotella, Lachnospira, Muribaculaceae; alloprevotella sp., Rikenellaceae sp., Butyricicoccus sp., ASF356 sp., Ruminiclostridium sp., Lactobacillus sp., Alistipes sp., Lachnoclostridium sp., Roseburia sp., Bacteroides sp., Prevotella sp., Lachnospiraceae sp., Muribaculaceae sp.*

In addition, as described above in the aforementioned embodiments, the co-culture experiments of LAB-EV with *Lactobacillus acidophilus,* as well as fluorescence staining experiments of LAB-EV and gut microorganisms, showed that LAB-EV was consumed by specific groups of microorganisms. Moreover, flow cytometry experiments revealed that approximately 32.31% of gut microorganisms utilized LAB-EV, and further sequencing showed that the two most abundant bacterial genera that consumed LAB-EV are *Muribaculaceae* and *Lachnospiraceae,* accounting for 22% and 19%, respectively.

### Embodiment 8: Identification of novel Pediococcus acidilactici strain of the present invention

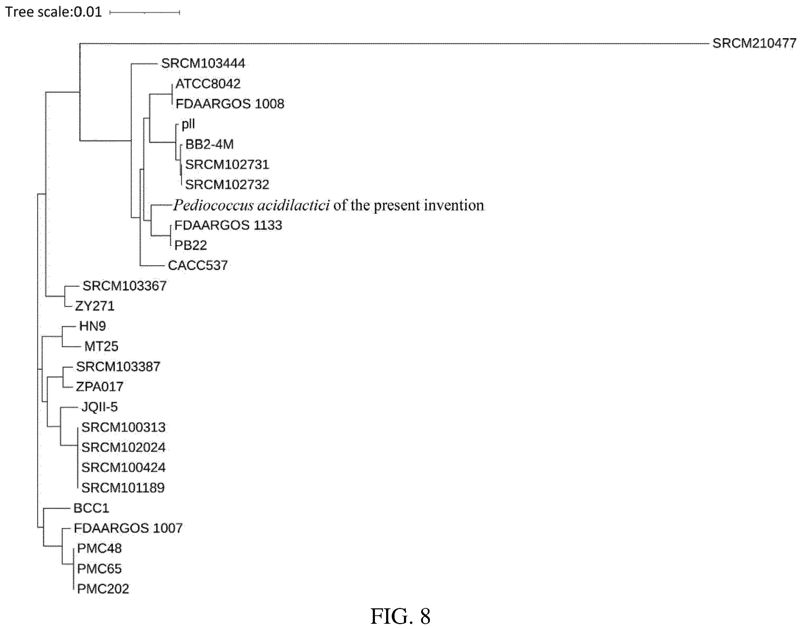

In the present embodiment, the complete genome sequence of *Pediococcus acidilactici* of the present invention was analyzed using the second-generation MiSeq (Illumina, Inc., USA) and the third-generation MinION (Oxford Nanopore Technologies, Inc., UK) sequencing platform. To identify differences, the genome was then compared with various genome databases, such as GenBank, Ensembl, BioCyc or RefSeq. The similarity between *Pediococcus acidilactici* of the present invention and 27 other fully assembled genomes of *Pediococcus acidilactici* in GenBank was analyzed. As shown in Table 1 below, FastANI v1.33 was used to calculate the average nucleotide identity (ANI) similarity with *Pediococcus acidilactici* of the present invention. Additionally, a phylogenetic tree was constructed using Roary v 3.11.2 and FastTree 2.1 to illustrate the phylogenetic relationships between different strains of *Pediococcus acidilactici,* as shown in FIG. *8**. Pediococcus acidilactici* PB22 was the most similar strain to *Pediococcus acidilactici* of the present invention, with an ANI of 99.16%. Moreover, no *Pediococcus acidilactici* strain was identified to have a completely identical genome to the novel strain *Pediococcus acidilactici* of the present invention.

**Table 1. Average nucleotide identity (ANI) between Pediococcus acidilactici of the present invention and other strains of Pediococcus acidilactici**

| **Strain name** | **GenBank assembly accession** | **ANI (%)** |
|---|---|---|
| PB22 | GCA_003957355.1 | 99.16 |
| FDAARGOS_1133 | GCA_016726765.1 | 99.15 |
| CACC537 | GCA_010092385.1 | 99.08 |
| ATCC8042 | GCA_0043 5 5265.1 | 98.99 |
| FDAARGOS_1008 | GCA_016128055.1 | 98.96 |
| SRCM103444 | GCA_004103635.1 | 98.89 |
| pll | GCA_016653595.1 | 98.73 |
| BB2-4M | GCA_023221555.1 | 98.69 |
| SRCM102732 | GCA_009913 895.1 | 98.67 |
| SRCM 102731 | GCA_009913875.1 | 98.63 |
| SRCM100313 | GCA_002173 595.1 | 97.37 |
| SRCM102024 | GCA_028621815.1 | 97.37 |
| SRCM101189 | GCA_002174215.1 | 97.36 |
| SRCM100424 | GCA_002173 575.1 | 97.35 |
| JQII-5 | GCA_006770685.1 | 97.35 |
| MT25 | GCA_020150035.1 | 97.25 |
| HN9 | GCA_14906145.1 | 97.24 |
| SRCM103367 | GCA_004102605.1 | 97.22 |
| ZY271 | GCA_019844055.1 | 97.17 |
| BCC1 | GCA_001922325.1 | 97.13 |
| SRCM103387 | GCA_004101585.1 | 97.12 |
| PMC48 | GCA_011604585.1 | 97.11 |
| PMC202 | GCA_019448175.1 | 97.08 |
| FDAARGOS_1007 | GCA_016127815.1 | 97.07 |
| PMC65 | GCA_013127755.1 | 97.04 |
| ZPA017 | GCA_001767275.1 | 97.02 |
| SRCM210477 | GCA_024970065.1 | 90.17 |

Next, to effectively identify the DNA sequences of *Pediococcus acidilactici* of the present invention, the present embodiment designed primers that can recognize genomic variation regions to differentiate different strains of the same species of *Pediococcus acidilactici.* The primers are listed in Table 2. Table 3 shows the primer combinations and the amplification sequences of the target strains. These sequences were compared with the RefSeq database, which includes complete genomes, scaffolds, and contigs. The amplification sequence of the primer combination CGPA01_1590-F/R, used to identify *Pediococcus acidilactici* of the present invention, was only present in the genome of the *Pediococcus acidilactici* strain MA18/5M. The amplification sequence for the primer combination MA18/5M_850-F/R was only present in MA18/5M and is absent in the genome of *Pediococcus acidilactici* of the present invention.

**Table 2. Primers with specific identification properties**

| Primer name and number | Sequence ID Number |
|---|---|
| CGPA01_1590-F | 1 |
| CGPA01_1590-R | 2 |
| MA18/5M_850-F | 3 |
| MA18/5M_850-R | 4 |

**Table 3. Sequences of amplified fragments of CGPA01_1590-F/R and MA18/5M_850-F/R primer combinations**

| Primer combinations | Target bacterial strain | Sequence length (bp) | Sequence ID Number |
|---|---|---|---|
| CGPA01_1590-F/R | *Pediococcus acidilactici* of the present invention | 115 | 5 |
| MA18/5M_850-F/R | MA18/5M | 505 | 6 |

Next, the primer combinations listed in Table 2 were used in Polymerase Chain Reaction (PCR). The PCR reaction had a total volume of 20 µl, including 0.1 ng of bacterial DNA, 500 nM of primers, and 10 µl of Taq DNA Polymerase Master Mix RED (Ampliqon A/S, DK). The reaction conditions for PCR were as follows: 95°C for 2 min, followed by 30 cycles of 95°C for 20 sec, annealing at 60°C for 30 sec, and extension at 72°C for 30 sec, with a final extension step of 5 min at 72°C. After completion of the PCR reaction, the PCR product was removed and subjected to electrophoresis on a 1.8% agarose gel in 1X TAE buffer at 100V for 40 minutes. The gel was then stained with SYBR Safe dye (Thermo Fisher Scientific, USA), and the stained gel was observed under a blue light (470 nm) transilluminator and photographed for record.

Table 4 lists the bacterial strains used to confirm the unique sequences of the *Pediococcus acidilactici* of the present invention. Except for the *Pediococcus acidilactici* of the present invention and MA18/5M, the listed strains were purchased from the Bioresource Collection and Research Center, Food Industry Research and Development Institute, Hsinchu, Taiwan, and these strains all belong to the genus *Pediococcus,* including *Pediococcus pentosaceus, Pediococcus parvulus,* and different strains of *Pediococcus acidilactici* of the same species. The *Pediococcus acidilactici* MA18/5M (also known as R1001) was isolated from the Jarro-Dophilus EPS product (Jarrow Formulas, Inc.).

**Table 4. Comparison of strains**

| Bacterial strain | Strain ID / BCRC accession number |
|---|---|
| *Pediococcus acidilactici* | *Pediococcus acidilactici* of the present invention |
| *Pediococcus acidilactici* | MA18/5M (R1001) |
| *Pediococcus acidilactici* | BCRC 11063 |
| *Pediococcus acidilactici* | BCRC 17599 |
| *Pediococcus acidilactici* | BCRC 80388 |
| *Pediococcus pentosaceus* | BCRC 10068 |
| *Pediococcus pentosaceus* | BCRC 11064 |
| *Pediococcus pentosaceus* | BCRC 12843 |
| *Pediococcus pentosaceus* | BCRC 14015 |
| *Pediococcus pentosaceus* | BCRC 14053 |
| *Pediococcus pentosaceus* | BCRC 14024 |
| *Pediococcus pentosaceus* | BCRC 14923 |
| *Pediococcus pentosaceus* | BCRC 80114 |
| *Pediococcus parvulus* | BCRC 12575 |

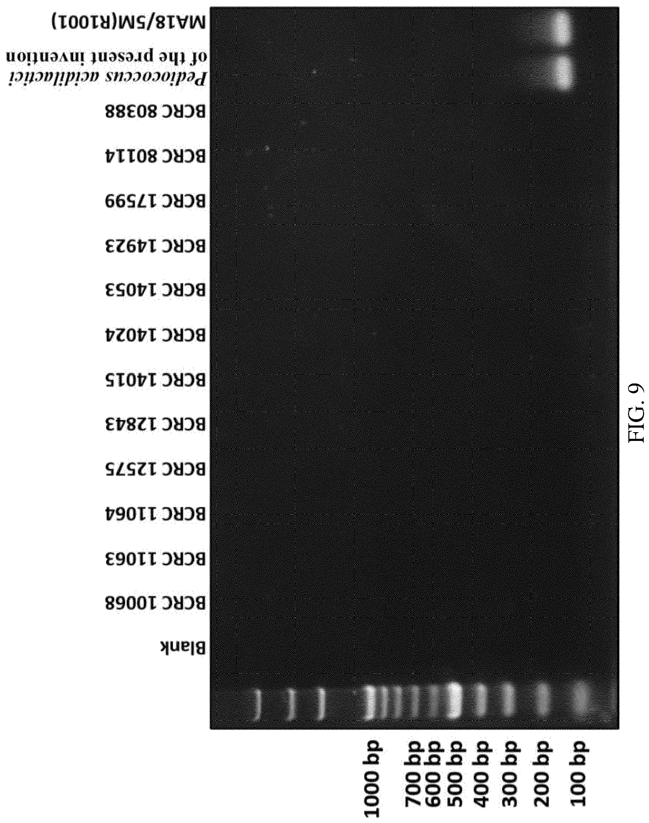

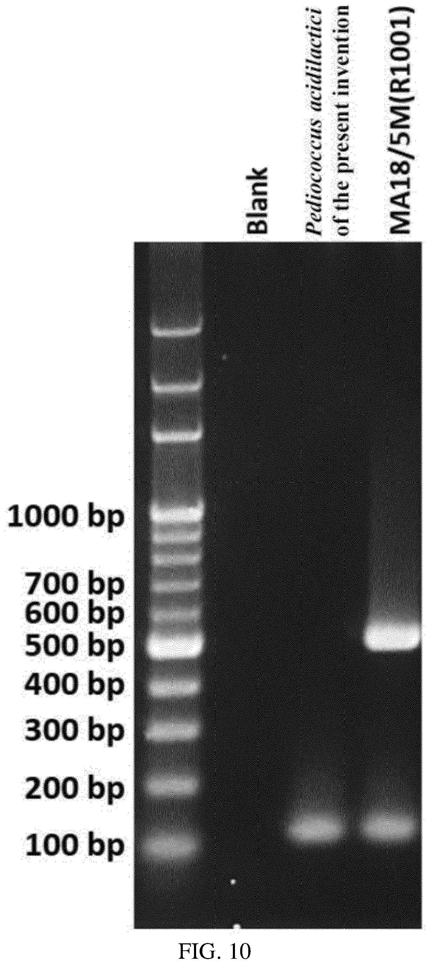

FIG. 9 shows the gel electrophoresis results of the PCR reactions using the CGPA01_1590-F/R primers and DNA samples from *Pediococcus* strains. As shown in FIG. 9, only *Pediococcus acidilactici* of the present invention and MA18/5M show a bright band at 115 bp. FIG. 10 shows the gel electrophoresis results of the PCR reactions using the MA18/5M_850-F/R primers and DNA samples from *Pediococcus acidilactici* of the present invention and MA18/5M. In the electrophoresis image, a bright band at 505 bp is observed only in *Pediococcus acidilactici* MA18/5M.

In summary, the genomic analysis revealed differences between the *Pediococcus acidilactici* of the present invention and other *Pediococcus acidilactic* strains, with the *Pediococcus acidilactic* PB22 genome showing the highest similarity to *Pediococcus acidilactici* of the present invention. Specific primers, including primer combinations CGPA01_1590-F/R and MA18/5M_850-F/R, were used in the PCR to differentiate *Pediococcus acidilactici* of the present invention from other publicly available *Pediococcus* strains. The CGPA01_1590-F/R primers amplified only *Pediococcus acidilactici* of the present invention and *Pediococcus acidilactici* MA18/5M, without amplifying other *Pediococcus* strains. Therefore, it is necessary to use the MA18/5M_850-F/R primers to further differentiate between *Pediococcus acidilactici* of the present invention and *Pediococcus acidilactici* MA18/5M. *Pediococcus acidilactici* of the present invention only showed a bright band that was amplified by the CGPA01_1590-F/R primers, while *Pediococcus acidilactici* MA18/5M showed bright bands that were amplified by CGPA01_1590-F/R primers and MA18/5M_850-F/R primers. Furthermore, based on the aforementioned genomic analysis and PCR results, it can be determined that the *Pediococcus acidilactici* SWP-CGPA01 provided by the present invention is a novel isolated lactic acid bacterial strain.

The above descriptions have fully and clearly illustrated the novel lactic acid bacterial strain, a composition for improving gut microbiota composition, products of said lactic acid bacterial strain, and a method for the preparation of the composition. It should be emphasized that the above descriptions are made on embodiments of the present invention; however, the embodiments are not intended to limit the scope of the present invention, and all equivalent implementations or alterations within the spirit of the present invention still fall within the scope of the present invention.

The *Pediococcus acidilactici* of the present invention is deposited at the National Collection of Industrial, Food and Marine Bacteria (NCIMB Ltd.) in the United Kingdom. The date of deposit is January 12, 2023, and the Accession number is NCIMB 44102.

## Claims

1. A lactic acid bacterial strain, which is a *Pediococcus acidilactici,* deposited at the NCIMB Ltd., with an Accession number of NCIMB 44102.

2. A composition for improving gut microbiota composition, comprising an effective amount of extracellular vesicles secreted by a lactic acid bacterial strain, wherein said lactic acid bacterial strain is a *Pediococcus acidilactici,* deposited at the NCIMB Ltd., with an Accession number of NCIMB 44102.

3. The composition of claim 2, wherein said effective amount is at least 10⁸ particles/ml of said extracellular vesicles.

4. The composition of claim 2, wherein said extracellular vesicles are selected from the group consisting of exosomes, microvesicles, ectosomes, and apoptotic bodies.

5. The composition of claim 2, wherein the composition is effective in improving the gut microbiota composition that affects the progression of Alzheimer's disease (AD).

6. The composition of claim 2, wherein said extracellular vesicles can affect the growth of *Firmicutes* and/or *Bacteroidetes.*

7. The composition of claim 2, wherein said extracellular vesicles can affect the growth of at least one bacterial class selected from the group consisting of *Bacteroidia, Clostridia,* and *Bacilli.*

8. The composition of claim 2, wherein said extracellular vesicles can affect the growth of at least one bacterial family selected from the group consisting of *Lactobacillaceae, Muribaculaceae, Lachnospiraceae, Clostridiaceae, Desulfovibrionaceae, Erysipelotrichaceae, Eggerthellaceae, Akkermansiaceae, Ruminococcaceae,* and *Eubacteriaceae.*

9. The composition of claim 2, wherein said extracellular vesicles can affect the growth of *Muribaculum* and/or *Lachnospira.*

10. The composition of claim 2, wherein said extracellular vesicles can facilitate the growth of *Lactobacillus acidophilus.*

11. A nutritional supplement comprising a *Pediococcus acidilactici,* deposited at the NCIMB Ltd., with an Accession number of NCIMB 44102.

12. A food product comprising a *Pediococcus acidilactici,* deposited at the NCIMB Ltd., with an Accession number of NCIMB 44102.

13. A dietary supplement comprising a *Pediococcus acidilactici,* deposited at the NCIMB Ltd., with an Accession number of NCIMB 44102.

14. A food additive comprising a *Pediococcus acidilactici,* deposited at the NCIMB Ltd., with an Accession number of NCIMB 44102.

15. A pharmaceutical composition comprising a *Pediococcus acidilactici,* deposited at the NCIMB Ltd., with an Accession number of NCIMB 44102.

16. A feedstuff comprising a *Pediococcus acidilactici,* deposited at the NCIMB Ltd., with an Accession number of NCIMB 44102.

17. A method for the preparation of a composition using a lactic acid bacterial strain and/or extracellular vesicles secreted by the lactic acid bacterial strain, wherein said lactic acid bacterial strain is a *Pediococcus acidilactici,* deposited at the NCIMB Ltd., with an Accession number of NCIMB 44102.
